# EUROPÄISCHE PATENTANMELDUNG

(11) **EP 4 678 794 A1**
(43) Veröffentlichungstag der Anmeldung: **14.01.2026**
(21) Anmeldenummer: 24188086.3
(22) Anmeldetag: 11.07.2024
(51) Int. Cl.: D01G 31/00, G01N 33/36

(54) **PRÜFEINRICHTUNG UND DEREN PRÜFVERFAHREN**

(71) Anmelder: Rieter AG, 8406 Winterthur (CH)
(72) Erfinder: SCHELLING, Patrick, 8487 Weisslingen (CH); BRAUN, Lukas, 8408 Winterthur (CH); WOLFER, Tobias, 8595 Altnau (CH); DÜGGELI, Jonas, 6312 Steinhausen (CH); LANTER, Joshua, 6403 Küssnacht (CH)
(74) Vertreter: Rieter

(57) **Zusammenfassung**

Die vorliegende Erfindung betrifft eine Prüfeinrichtung (100) zum Prüfen von Fasermaterial (990) auf dessen Kennwerte. Die Prüfeinrichtung umfasst eine Sammelfläche (110), die das von aus einer Mündung tretende Fasermaterialen (990) sammelt, eine Andruckfläche (120), und wenigstens einen Stellmotor (131, 132), der wenigstens eine der Flächen (110, 120) bewegt, um das gesammelte Fasermaterial zu drücken und die Eigenschaften des gesammelten Fasermaterials zu erfassen.

## Beschreibung

### Technisches Gebiet

Die vorliegende Erfindung betrifft eine Einrichtung und ein Verfahren zur Analyse von Komponenten einer Fasermasse, wobei die Komponenten der Fasermasse Fasern, Teile von Fasern, Trash, Staub, Pflanzenrückstände und andere Verschmutzungen umfassen können.

### Technologischer Hintergrund

Naturfasern wie Baumwolle können durch Nicht-Primärfasermaterial verunreinigt sein, das oft allgemein als Abfall bezeichnet wird. Solche Verunreinigungen können z. B. Schalen, Samen, Zweige, Rinde, Blätter, Schmutz oder Steine sein. Die Messung des Nicht-Faseranteils einer Faserprobe erfolgt im Stand der Technik durch Trennen der Fasern in einer Faserprobe bzw. Fasermasse von einem möglichst grossen Teil des Nicht-Faseranteils in der Faserprobe und durch Wiegen oder anderweitiges Quantifizieren von den Fasern und dem Abfall, die von der ursprünglichen Faserprobe abgetrennt wurden. Bei den verwendeten Separatoren bleiben mehr oder weniger Mengen an Fasern mit dem abgetrennten Abfall vermischt, was es schwierig macht, die Eigenschaften des gesammelten Fasermaterials zu erfassen.

Aus dem Stand der Technik ist eine Trennvorrichtung bekannt, mit der versucht wird, den Abfall besser von den Fasern trennen zu können. Die Faserprobe wird dabei auf eine Oberfläche eines Trennzylinders aufgebracht, wobei der Trennzylinder in einer Drehrichtung rotiert und eine zylindrische Oberfläche mit Stiften aufweist. Fasern der Faserprobe werden mit dem Trennzylinder in Eingriff gebracht und an der Oberfläche des Trennzylinders zurückgehalten. Die auf der Sammelfläche zurückgehaltenen Fasern können mit einem Gerät visuell erfasst.

Nachteilig hierbei ist es, dass eine genauere quantitative Analyse oder gar eine qualitative Analyse der in der Fasermasse enthaltenen Fasern nicht möglich ist, weil die Fasern nicht von extern Einfluss geschützt sind und die Eigenschaften des Fasermaterials, wie z.B. die Struktur der Faser, nicht analysiert werden kann.

Aufgabe der vorliegenden Erfindung ist es, die aus dem Stand der Technik bekannten Nachteile zu beseitigen und insbesondere eine quantitative und eine qualitative Analyse der Komponenten der vorgelegten Fasermasse durchführen zu können.

### Zusammenfassung der Erfindung

Die Aufgabe wird durch die Merkmale der Erfindung ganz oder teilweise gelöst. Zur Lösung der Aufgabe wird eine Prüfeinrichtung zum Prüfen von Fasermaterial auf dessen Kennwerte vorgeschlagen. Fasermaterialen können Fasern, Teile von Fasern und Verunreinigungen, wie Trash, Staub, Pflanzenrückstände und andere Verschmutzungen umfassen, und können, beispielsweise einem sogenannten Streckenband, Kardenband oder Teilen davon, mittels einer sich drehenden Speisewalze einer drehenden Auflösewalze zugeführt werden. Die Prüfeinrichtung umfasst:
- eine Sammelfläche; die Sammelfläche ist so ausgelegt, dass sie Fasermaterial sammelt;
- eine Andruckfläche; die Andruckfläche ist so ausgelegt, dass das gesammelte Fasermaterial gedrückt ist, um die Eigenschaften des gesammelten Fasermaterials zu erfassen;
- wenigstens einen Stellmotor; der wenigstens eine Stellmotor ist so ausgelegt, dass er wenigstens eine Fläche, aus Sammelfläche und Andruckfläche, zwischen wenigstens einer ersten Position, wobei das gesammelte Fasermaterial zwischen der Andruckfläche und der Sammelfläche gedrückt ist, und wenigstens einer zweiten Position, wobei das gesammelte Fasermaterial befreit wird und von Prüfeinrichtung teilweise oder ganz entfernt wird, bewegt.

Dank der vorliegenden Erfindung können zumindest die zwischen der Sammelfläche und der Andruckfläche gedrückten Fasern erfasst werden und hinsichtlich ihrer Art und Beschaffenheit analysiert werden. Durch das zwischen der Sammelfläche und der Andruckfläche Drücken ist das gesammelte Fasermaterial von extern Einfluss geschützt und kann nicht kontaminiert werden durch in der Luft herumschwebenden Fasern oder anderen Schmutz, die nicht aus der Probe stammen würde. Durch die vereinzelten, ausgebreiteten Fasern auf der Sammelfläche können die Fasern einzeln erkannt werden, und kann z.B. die Struktur der Faser und/oder die Länge der Faser besser analysierte werden, weil die Fasern zwischen der Sammelfläche und der Andruckfläche gedrückt sind. So kann beispielsweise ermittelt werden, ob, welche und wie viele Fasern auf der Sammelfläche oder in einem bestimmten Abschnitt der Sammelfläche liegen, und deren Struktur. Die Fasern liegen dabei vorzugsweise beabstandet voneinander oder mit nur wenig Überlappung.

Gemäss einer Ausführungsform umfasst der wenigstens einen Stellmotor, wenigstens einen ersten Stellmotor, der konfiguriert ist, die Sammelfläche zu bewegen, und/oder wenigstens einen zweiten Stellmotor, der konfiguriert ist, die Andruckfläche zu bewegen.

Dank des wenigstens einen ersten Stellmotors und des wenigstens einen zweiten Stellmotors, können die Sammelfläche und/oder die Andruckfläche einzeln bewegt werden, miteinander bewegt werden, zueinander bewegt werden und/oder koordiniert bewegt werden.

Gemäss einer Ausführungsform umfasst die Prüfeinrichtung wenigstens eine Putzeinrichtung, die wenigstens eine Putzeinrichtung so ausgelegt ist, dass sie die wenigstens eine Fläche, aus Sammelfläche und Andruckfläche, putzt, vorzugsweise, wenn die wenigstens eine Fläche, aus Sammelfläche und Andruckfläche, zwischen der wenigstens einen ersten Position und der wenigstens einen zweiten Position bewegt wird.

Gemäss einer Ausführungsform umfasst die wenigstens eine Putzeinrichtung wenigstens eine erste Putzeinrichtung und/oder wenigstens eine zweite Putzeinrichtung, wobei die wenigstens eine erste Putzeinrichtung so konfiguriert ist, um die Sammelfläche zu putzen, und wobei die wenigstens eine zweite Putzeinrichtung so konfiguriert ist, um die Andruckfläche zu putzen.

Dank der Putzeinrichtung können die wenigstens eine Fläche, aus Sammelfläche und Andruckfläche, von Fasern befreit werden, und vorzugsweise, wenn die wenigstens eine Fläche bewegt ist.

Gemäss einer Ausführungsform ist die Sammelfläche eine Platte oder ein Riemen, und/oder ist die Andruckfläche ein Riemen oder eine Platte.

Vorteilhaft kann der Riemen die zu erfassenden Fasern zwischen der wenigstens einen ersten Position und der wenigstens einen zweiten Position bewegt werden und/oder kann die Sammelfläche und/oder die Andruckfläche die zu erfassenden Fasern besser drücken.

Gemäss einer Ausführungsform umfasst die Sammelfläche und/oder die Andruckfläche teilweise oder ganz eine Schicht aus geringen Reibungskoeffizienten, vorzugsweise aus Polytetrafluorethylen.

Gemäss einer Ausführungsform besteht die Sammelfläche und/oder die Andruckfläche teilweise oder ganz aus Stahl, vorzugsweise aus Edelstahl.

Dank einer der Ausführungsformen kann die Sammelfläche und/oder die Andruckfläche teilweise oder ganz mit einer Schicht aus geringen Reibungskoeffizienten, vorzugsweise aus Polytetrafluorethylen, und/oder aus Stahl, vorzugsweise aus Edelstahl, leicht von den Fasern befreit werden.

Gemäss einer Ausführungsform umfasst die Sammelfläche ganz oder teilweise einen transparenten Bereich zum Erfassen der Eigenschaften des gesammelten Fasermaterials.

Vorteilhaft können die Eigenschaften des gesammelten Fasermaterials erfasst werden.

Gemäss einer Ausführungsform umfasst die Prüfeinrichtung eine Analyseeinrichtung, die konfiguriert ist, die die Eigenschaften des gesammelten Fasermaterials zu erfassen, vorzugsweise in der wenigstens einen ersten Position.

Vorteilhaft können die Eigenschaften des gesammelten Fasermaterials automatisch erfasst werden.

Gemäss einer Ausführungsform umfasst die Analyseeinrichtung einen Sender und/oder einen Empfänger, wobei der Sender so ausgelegt ist, dass er das gesammelte Fasermaterial strahlt, und/oder der Empfänger so ausgelegt ist, dass er teilweise oder ganz die reflektierten elektromagnetischen Wellen und/oder die durchgedrungenen elektromagnetischen Wellen empfängt.

Gemäss einer Ausführungsform umfasst die Analyseeinrichtung eine Kamera, ein Ultraschallgerät oder ein Röntgengerät.

Dank dieser Konfiguration verfügt die Analyseeinrichtung einen breiten Frequenzbereich, um zu analysieren.

Die Aufgabe wird durch die Merkmale der Erfindung ganz oder teilweise gelöst. Zur Lösung der Aufgabe wird ein Prüfverfahren zum Prüfen von Fasermaterial auf dessen Kennwerte vorgeschlagen. Das Prüfverfahren umfasst:
- eine Sammlung von Fasermaterialen auf einer Sammelfläche;
- einen Druck des gesammelten Fasermaterials zwischen einer Andruckfläche und der Sammelfläche;
- eine Erfassung der Eigenschaften des gesammelten Fasermaterials; und,
- eine Befreiung der Sammelfläche und der Andruckfläche von Fasermaterial.

Dank des vorliegenden Verfahrens können zumindest einige der zwischen der Sammelfläche und der Andruckfläche gedrückten Fasern erfasst und hinsichtlich ihrer Art und Beschaffenheit analysiert werden. Durch das zwischen der Sammelfläche und der Andruckfläche Drücken ist das gesammelte Fasermaterial von extern Einfluss geschützt und kann nicht kontaminiert werden durch in der Luft herumschwebenden Fasern oder anderen Schmutz, die nicht aus der Probe stammen würde. Durch die vereinzelten, ausgebreiteten Fasern auf der Sammelfläche können die Fasern einzeln erkannt werden, und kann z.B. die Struktur der Faser und/oder die Länge der Faser besser analysierte werden, weil die Fasern zwischen der Sammelfläche und der Andruckfläche gedrückt sind, was nicht der Fall ist, wenn die Fasern auf einer Saugfläche sind, weil die Fasern nur Punktberührungen mit der Saugfläche und kein Linienberührungen oder Flächenberührung. So kann beispielsweise ermittelt werden, ob, welche und wie viele Fasern auf der Sammelfläche oder in einem bestimmten Abschnitt der Sammelfläche liegen, und deren Struktur. Die Fasern liegen dabei vorzugsweise beabstandet voneinander oder mit nur wenig Überlappung.

Dank einer der Ausführungsformen umfasst das Prüfverfahren eine Bewegung der wenigstens einen Fläche, aus Sammelfläche und Andruckfläche, zwischen wenigstens einer ersten Position, wobei das gesammelte Fasermaterial zwischen der Andruckfläche und der Sammelfläche gedrückt ist, und wenigstens einer zweiten Position, wobei die Sammelfläche und die Andruckfläche vom gesammelten Fasermaterial befreit werden.

Dank der Bewegung der Sammelfläche und/oder der Andruckfläche kann das gesammelte Fasermaterial in einer Position gedrückt werden und in der anderen Position von der Prüfeinrichtung befreit werden.

Gemäss einer Ausführungsform findet die Erfassung statt, während der in wenigstens einer ersten Position sich die wenigstens eine Fläche befindet.

Vorteilhaft kann die Art und die Beschaffenheit des gesammelten Fasermaterials analysiert werden.

Gemäss einer Ausführungsform findet die Befreiung statt, während der in wenigstens einer zweiten Position sich die wenigstens eine Fläche befindet.

Vorteilhaft kann die Prüfeinrichtung von dem gesammelten Fasermaterial befreit und bereit für die nächste Analyse sein, ohne dass sie mit schon erfasstem Fasermaterial kontaminiert ist.

### Beschreibung der Figuren

Die vorstehenden und weiteren Ziele, Merkmale, Aspekte und Vorteile der Erfindung werden aus der folgenden detaillierten Beschreibung der Ausführungsformen ersichtlich, die unter Bezugnahme auf die beigefügten Zeichnungen illustrativ und nicht einschränkend dargestellt sind, wobei
- die Figuren 1 und 2 eine Sammlung **510** von Fasermaterialen **990** und einen Druck des gesammelten Fasermaterials **990** zwischen einer Andruckfläche **120** und der Sammelfläche **110** gemäss einer Ausführungsform;
- die Figuren 3-6 das Prüfverfahren, das von unterschiedlichen Ausführungsformen durchgeführt ist, darstellen; und,
- Die Figur 7 ein Ausführungsform mit dem Prüfverfahren gemäss der Erfindung.

Bei der nachfolgenden Beschreibung der dargestellten Ausführungsbeispiele werden für Merkmale, die in ihrer Ausgestaltung, und/oder Wirkweise identisch, und/oder zumindest vergleichbar sind, gleiche Bezugszeichen verwendet, auch wenn sie in unterschiedlichen Ausführungsbeispielen gezeigt sind. Sofern diese nicht nochmals detailliert erläutert werden, entspricht deren Ausgestaltung, und/oder Wirkweise der Ausgestaltung und Wirkweise der vorstehend bereits beschriebenen Merkmale.

### Beschreibung einer Ausführungsformen

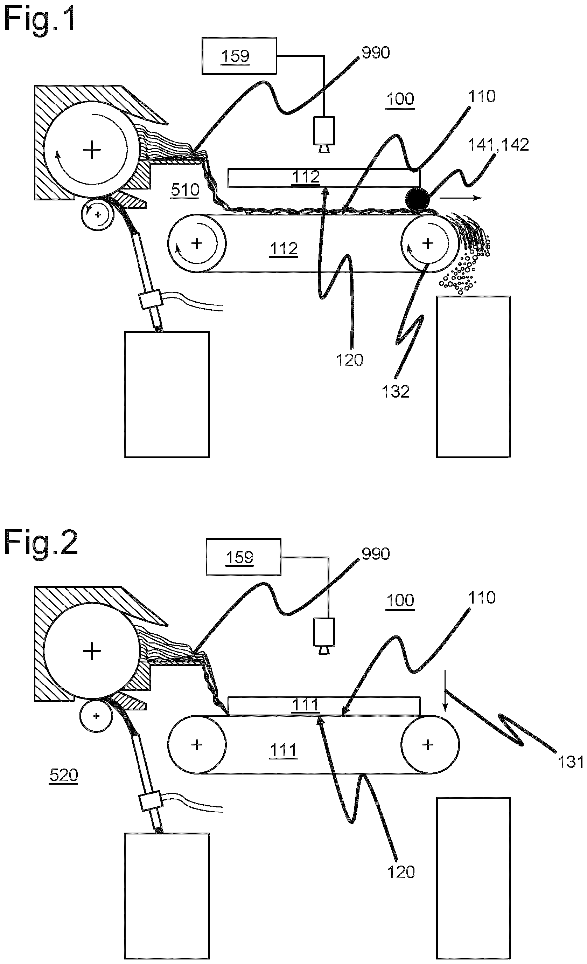

Figur 1 zeigt eine Seitenansicht einer erfindungsgemässen Prüfeinrichtung **100** zur Analyse und Prüfen von Fasermaterial **990** einer Fasermasse. Komponenten **990** bzw. Fasermaterialen **990** der Fasermasse können Fasern, Teile von Fasern und Verunreinigungen, wie Trash, Staub, Pflanzenrückstände und andere Verschmutzungen umfassen.

Die Zuführung der Fasermasse in eine Speisewalze erfolgt mit einer Absaugeinrichtung. Die Absaugeinrichtung wird in die Nähe der Fasermasse, welche sich in einer Spinnkanne befindet, gebracht und saugt die Fasermasse aus der Spinnkanne ab. Die Fasermasse wird dabei durch ein Saugrohr geführt und in den Bereich der Speisewalze gebracht. Eine Saugströmung in dem Saugrohr wird in dem vorliegenden Ausführungsbeispiel durch eine Coanda-Düse erzeugt, indem Luft in die Coanda-Düse eingeblasen und damit die Saugströmung in dem Saugrohr erzeugt wird. Die Saugströmung zieht die Fasermasse aus der Spinnkanne ab und fördert sie bis zur Speisewalze. Nachdem genügend Fasermasse entnommen wurde, wird die Fasermasse getrennt und die Absaugeinrichtung kann für eine weitere Absaugung verwendet werden.

Die Absaugeinrichtung kann entweder stationär an der Vorrichtung angeordnet sein und die Fasermasse wird in den Bereich der Absaugeinrichtung gebracht, beispielsweise indem die Spinnkanne unter die Absaugeinrichtung gestellt wird. Sie kann aber auch mobil sein, was bedeutet, dass die Absaugeinrichtung je nach Bedarf in die Nähe der Fasermasse an einer Maschine einer Spinnereianlage gebracht wird und dort zusammen mit der Vorrichtung eine Analyse der Fasermasse durchführt.

Um die Komponenten **990** analysieren und prüfen zu können, können sie vereinzelt und/oder ausgebreitet werden. Hierzu können die Fasern der Fasermasse, beispielsweise einem sogenannten Streckenband, Kardenband oder Teilen davon, mittels einer sich drehenden Speisewalze einer drehenden Auflösewalze zugeführt werden. Die Auflösewalze kann in einem Auflösewalzengehäuse angeordnet sein. Die Speisewalze kann sich an einer Einspeiseöffnung des Auflösewalzengehäuses befinden. Durch die Drehung der Speisewalze und der Auflösewalze kann die Fasermasse in den Bereich der Auflösewalze eingeführt werden und das Fasermaterial **990** der Fasermasse können durch Zähne oder Nadeln, welche am Umfang der Auflösewalze angeordnet sind, vereinzelt oder ausgebreitet werden. Verunreinigungen, welche sich in der Fasermasse befinden, können an einer Schmutzausscheideöffnung ausgeschieden werden. Die Fasern selbst können am Umfang der sich drehenden Auflösewalze haften bleiben und können beschleunigt werden. Die Auflösewalze kann einen spiralförmig auf dem Umfang der Auflösewalze verlaufenden Sägezahndraht aufweisen. Der Sägezahndraht kann dafür sorgen, dass die Fasern im Bereich der Einspeiseöffnung erfasst **530** und beschleunigt werden können.

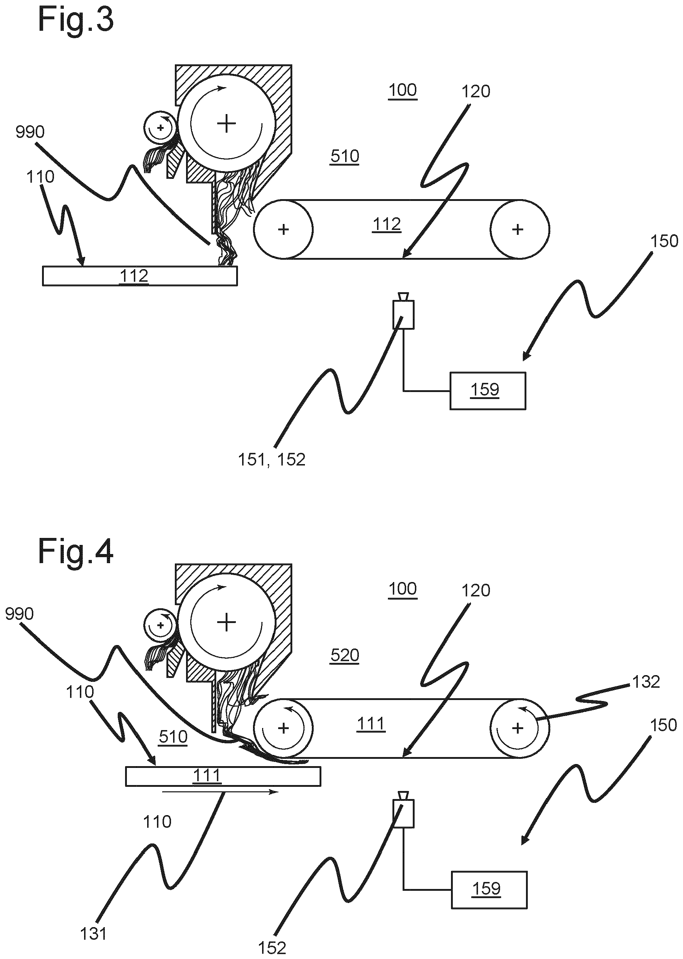

Nachdem die Fasern eine bestimmte Geschwindigkeit erreicht haben, können sie sich im Bereich einer Austrittsöffnung des Auflösewalzengehäuses von der sich drehenden Auflösewalze lösen, aufgrund der einwirkenden Zentrifugalkraft ab. Die Fasern können dabei in einen Speisekanal gelangen und können in einer Mündung des Speisekanals aus dem Speisekanal austreten. Anschliessend können die Fasern auf die erfindungsgemässe Prüfeinrichtung **100** treffen, beziehungsweise auf eine Sammelfläche **110** der Prüfeinrichtung **100.** Die Sammelfläche **110** kann so ausgelegt sein, dass sie das aus der Mündung tretendem Fasermaterial **990** sammeln kann, wie z.B. in Figs. 1 & 3 dargestellt ist.

Damit die Fasern, welche die Auflösewalze im Bereich der Austrittsöffnung verlassen können, kann die Mündung derart ausgestaltet sein, dass sie nicht besaugt ist. Die Mündung kann somit nicht in unmittelbarer Nähe und Wirkung der Besaugung durch den Saugkanal sein. Die in dem Speisekanal befindlichen Fasern können sich somit von der Auflösewalze ohne Einwirkung von Unterdruck lösen. Damit ist gewährleistet, dass die Fasern weitgehend vereinzelt von der Auflösewalze gelöst werden können und auf die Sammelfläche **110** gelangen.

Damit die Fasern sicher auf der Sammelfläche **110** liegen bleiben können, kann die Prüfeinrichtung **100** eine Andruckfläche **120** umfassen, wie z.B. in Figs. 2 & 4. Ausführungsgemäss kann die Andruckfläche **120** so ausgelegt sein, dass das gesammelte Fasermaterial **990** gedrückt **520** sein kann, um die Eigenschaften des gesammelten Fasermaterials **990** besser erfassen **530** zu können. So können die zwischen der Sammelfläche **110** und der Andruckfläche **120** gedrückten Fasern erfasst **530** werden und hinsichtlich ihrer Art und Beschaffenheit analysiert werden. Durch das zwischen der Sammelfläche **110** und der Andruckfläche **120** Drücken ist das gesammelte Fasermaterial **990** von extern Einfluss geschützt, weil keine in der Luft herumschwebenden Fasern oder kein Schmutz und kann nicht kontaminiert werden durch, die nicht aus der Probe stammen würde.

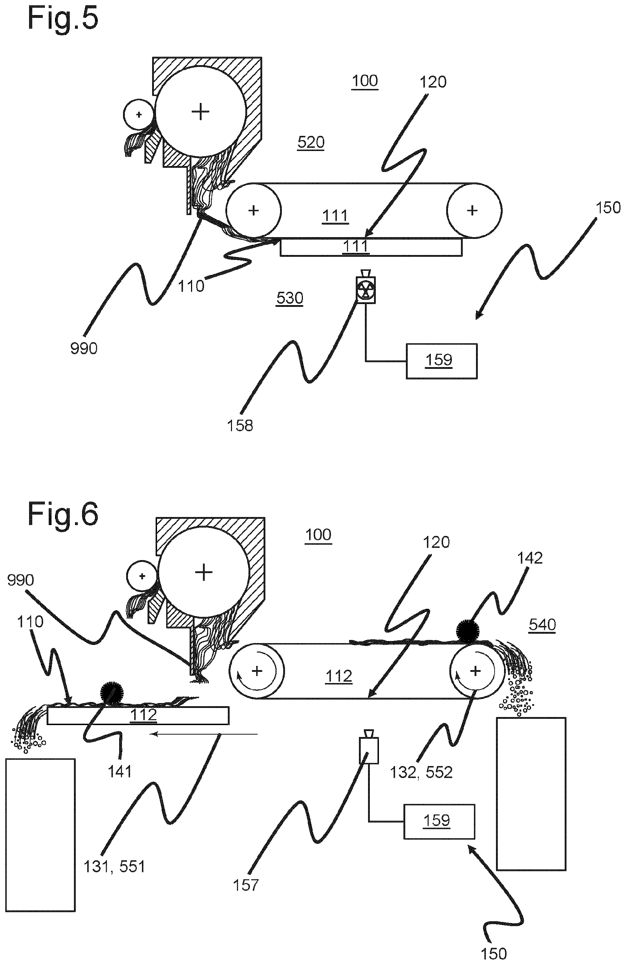

Durch die vereinzelten, ausgebreiteten Fasern auf der Sammelfläche **110** können die Fasern einzeln erkannt werden, und kann z.B. die Struktur der Faser und/oder die Länge der Faser besser analysierte werden, wie in Figs. 2 & 5, weil die Fasern zwischen der Sammelfläche **110** und der Andruckfläche **120** gedrückt sind. So kann beispielsweise ermittelt werden, ob, welche und wie viele Fasern auf der Sammelfläche **110** oder in einem bestimmten Abschnitt der Sammelfläche **110** liegen und deren Struktur. Die Fasern liegen dabei vorzugsweise beabstandet voneinander oder mit nur wenig Überlappung.

Durch die vereinzelten, ausgebreiteten Fasern auf der Sammelfläche **110** können die Fasern einzeln erkannt werden. Ferner kann beispielsweise ermittelt werden, ob, welche und wie viele Fasern auf der Sammelfläche **110** oder in einem bestimmten Abschnitt der Sammelfläche **110** liegen. Die Fasern liegen dabei vorzugsweise beabstandet voneinander oder mit nur wenig Überlappung.

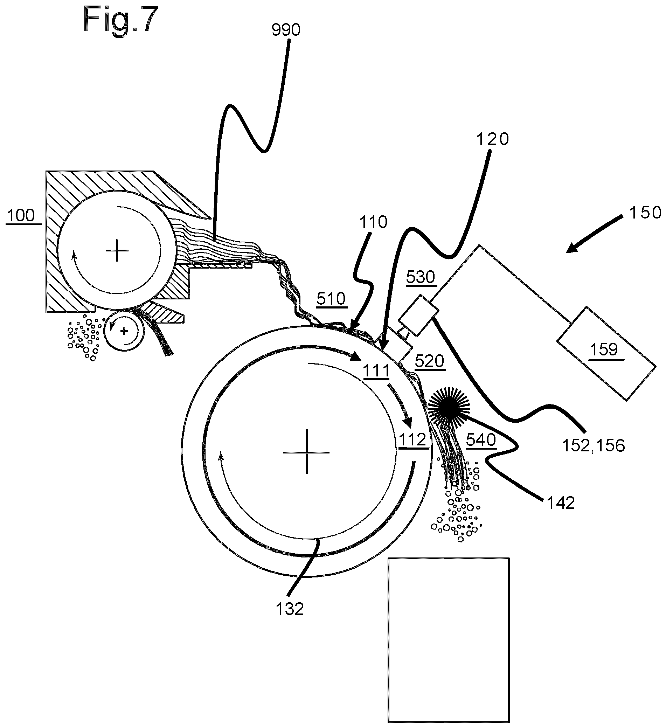

Tatsächlich können die Fasern vereinzelt werden, d. h. mit Abstand zueinander bzw. mit möglichst wenig Überlappungen auf der Sammelfläche **110** abgelegt. Am Ende der Erfassung **530** der Eigenschaften des gesammelten Fasermaterials **990** können die Fasern von der Sammelfläche **110** und der Andruckfläche **120** entfernt **540** werden und an einer Entnahmestelle verlassen, wie in Figs. 1, 6 & 7. Wie in Fig. 1 gezeigt, können die Fasern sich lösen, wenn sie zusammen mit der sich drehenden Sammelfläche **110** bis an das Ende transportiert wurden und fallen in einem Behälter. Ähnliches ist mit der Ausführungsform der Fig. 7, wobei die Fasern sich lösen können, wenn sie zusammen mit der sich drehenden Sammelfläche **110** und nach der Andruckfläche **120** transportiert wurden und fallen in einem Behälter.

Die an der Fasernausscheideöffnung des Auflösewalzengehäuses ausgeschiedenen Fasern fallen auf die Sammelfläche **110,** die eine Platte oder ein Riemen sein kann. Beziehungsweise kann die Andruckfläche **120** in Form einem Riemen oder einer Platte vorliegen. So kann der Riemen die zu erfassenden Fasern zwischen der wenigstens einen ersten Position **111** und der wenigstens einen zweiten Position **112** bewegt **551, 552** werden und/oder kann die wenigstens eine Fläche **110, 120,** aus Sammelfläche **110** und Andruckfläche **120,** die zu erfassenden Fasern besser drücken **520** so dass die Fasern auf der Sammelfläche **110** liegen bleiben und dort analysiert werden können.

Die Analyse des gesammelten Fasermaterials **990** erfolgt mit einer Analyseeinrichtung **150,** vorzugsweise in der wenigstens einen ersten Position **111.** Je nach Ausführungsform, kann die Analyseeinrichtung **150** einen Sender **151** und/oder einen Empfänger **152** umfassen. Der Sender **151** kann das gesammelte Fasermaterial **990** strahlen, und kann z.B. eine Lichtquelle sein. Der Empfänger **152** kann teilweise oder ganz die reflektierten elektromagnetischen Wellen und/oder die durchgedrungenen elektromagnetischen Wellen empfangen. Gemäss einer Ausführungsform kann die Analyseeinrichtung **150** eine Kamera **156** umfassen und, in diesem Fall, kann die Analyseeinrichtung **150** nur einen Empfänger **152** umfassen, weil das Umgebungslicht ausreichend kann und eine Lichtquelle entfallen kann. Gemäss anderen Ausführungsformen kann die Analyseeinrichtung **150** einen Sender **151** und einen Empfänger **152** umfassen, wie z.B. mit einem Ultraschallgerät **157** oder einem Röntgengerät **158.**

Im Fall einer Kamera **156** kann die optische Analyseeinrichtung **150** in einer elektronischen Datenverarbeitungsanlage **159,** beispielsweise einem Computer **159,** stattfinden. In dem Computer **159** können Signale, welche von den Kameras **156** geliefert werden können, ausgewertet werden. So können Bilder, welche von den Kameras **156,** vorzugsweise in der wenigstens einen ersten Position **111,** gemacht sind, beispielsweise mit Bildern einer Datenbank verglichen (nicht dargestellt) werden. Hieraus kann festgestellt werden, welcher Art die Fasern sein können. Ausserdem kann festgestellt werden, welche Länge, die auf der Sammelfläche **110** oder aufliegenden Fasern haben, um ermitteln zu können, ob es sich um komplette Fasern oder nur um Teile von Fasern handelt, die Länge der Fasern und/oder die Struktur der Fasern. Auch die Anzahl der festgestellten Fasern kann ermittelt werden, da die Fasern und die Verunreinigungen vereinzelt auf der Sammelfläche **110** liegen. Aus den Informationen, welche der Computer **159** aus den Aufnahmen der Kameras **156** entnehmen kann, ist ein Rückschluss auf die Zusammensetzung der Fasermasse zu erhalten. Hieraus kann wiederum die Weiterverarbeitung des Fasermaterials **990** sehr zielgerichtet erfolgen. Beispielsweise kann sich daraus ergeben, dass die Fasermasse einer besonderen Reinigung unterzogen werden muss, eine bestimmte Mischung mit anderen Fasermassen vorgenommen werden muss oder sie nur für das Verspinnen auf bestimmten Maschinen geeignet ist.

Nachdem sowohl die Fasern als auch die Verunreinigungen auf der Sammelfläche **110** zu liegen kommen, kann eine einzige Kamera **156** der optischen Analyseeinrichtung **157** ausreichend sein, um alle Komponenten **990** des Fasermaterials **990** erfassen **530** zu können und die entsprechenden Daten für eine Auswertung an den Computer **159** senden zu können. Für eine besonders gute Auswertung und Analyse der Komponenten **990** des Fasermaterials **990** kann es sein, dass die Fasern und die Verunreinigungen möglichst vereinzelt auf der Sammelfläche **110** liegen.

Die Kamera **156** der optischen Analyseeinrichtung **150** kann auf die Seite der Sammelfläche **110** gerichtet sein und kann dort die Fasern und die Verschmutzungen optisch erfassen **530,** weil die Sammelfläche **110,** die eine Platte oder ein Riemen sein kann, ganz oder teilweise einen transparenten Bereich für die vom Sender **151** verwendeten Wellenlängen umfassen kann, so kann die Analyseeinrichtung **150** die in der vorgelegten Fasermasse enthaltenen unterschiedlichen Komponenten **990** analysieren, dank eines breiten Frequenzbereichs bzw. eines unterschiedlichen Frequenzbereichs. Das gleich gilt auch für die Andruckfläche **120,** die in Form einem Riemen oder einer Platte vorliegen kann, und für ein Ultraschallgerät **157** oder ein Röntgengerät **158,** wie in Figs. 5 & 6.

Die entsprechenden Signale bzw. Bilder werden an den Computer **159** gesandt, wo sie ausgewertet werden, um die in der vorgelegten Fasermasse enthaltenen Komponenten **990** analysieren zu können.

Um die wenigstens eine Fläche **110, 120,** aus Sammelfläche **110** und Andruckfläche **120,** anzutreiben, kann die Prüfeinrichtung **100** wenigstens einen Stellmotor **131, 132** umfassen. Genauer kann der wenigstens eine Stellmotor **131, 132** wenigstens einen ersten Stellmotor **131,** der die Sammelfläche **110** bewegen **551** kann, und/oder wenigstens einen zweiten Stellmotor **132,** der die Andruckfläche **120** bewegen **552** kann, so dass die Sammelfläche **110** und/oder Andruckfläche **120** einzeln bewegt **551, 552** werden können, miteinander bewegt **551, 552** werden können, zueinander bewegt **551, 552** werden können und/oder koordiniert bewegt **551, 552** werden können, zwischen wenigstens einer ersten Position **111,** wobei das gesammelte Fasermaterial **990** zwischen der Andruckfläche **120** und der Sammelfläche **110** gedrückt **520** sein kann, und wenigstens einer zweiten Position **112,** wobei das gesammelte Fasermaterial **990** befreit **540** werden kann und von Prüfeinrichtung **100** teilweise oder ganz entfernt werden kann. Anders gesagt, findet vorzugsweise die Erfassung **530** statt, während der in wenigstens einer ersten Position **111** sich die wenigstens eine Fläche **110, 120** befindet, so dass die Art und die Beschaffenheit des gesammelten Fasermaterials **990** analysiert werden können, und findet vorzugsweise die Befreiung **540** statt, während der in wenigstens einer zweiten Position **112** sich die wenigstens eine Fläche **110, 120** befindet, so dass die Prüfeinrichtung **100** von dem gesammelten Fasermaterial **990** befreit **540** werden kann und bereit für die nächste Analyse, ohne dass sie mit schon erfasstem Fasermaterial **990** verseucht ist.

Was die Befreiung **540** der Sammelfläche **110** und der Andruckfläche **120** von Fasermaterial **990** betrifft, umfasst die Prüfeinrichtung **100** wenigstens eine Putzeinrichtung **141, 142,** dass sie die wenigstens eine Fläche **110, 120,** aus Sammelfläche **110** und Andruckfläche **120,** putzen **540** kann, vorzugsweise, wenn die wenigstens eine Fläche **110, 120,** aus Sammelfläche **110** und Andruckfläche **120,** zwischen der wenigstens einen ersten Position **111** und der wenigstens einen zweiten Position **112** bewegt **551, 552** werden kann. Beispielweise kann die wenigstens eine Putzeinrichtung **141, 142** wenigstens eine erste Putzeinrichtung **141** und/oder wenigstens eine zweite Putzeinrichtung **142** umfassen, wobei die wenigstens eine erste Putzeinrichtung **141** so konfiguriert ist, um die Sammelfläche **110** zu putzen **540,** und wobei die wenigstens eine zweite Putzeinrichtung **142** so konfiguriert ist, um die Andruckfläche **120** zu putzen **540,** so dass die wenigstens eine Fläche **110, 120,** aus Sammelfläche **110** und Andruckfläche **120,** von Fasern befreit **540** werden können, und vorzugsweise, wenn die wenigstens eine Fläche **110, 120** bewegt **551, 552** sein kann, wie in Figs. 1, 6 & 7 dargestellt ist.

Um die Befreiung **540** der Sammelfläche **110** und der Andruckfläche **120** erleichtern zu können, kann die Sammelfläche **110** und/oder die Andruckfläche **120** teilweise oder ganz eine Schicht aus geringen Reibungskoeffizienten, vorzugsweise aus Polytetrafluorethylen, umfassen. Im Gegensatz kann die Andruckfläche **120** und/oder die Sammelfläche **110** teilweise oder ganz aus Stahl, vorzugsweise aus Edelstahl, bestehen. Anders ausgedrückt kann die Andruckfläche **120** und/oder die Sammelfläche **110** einen Bereich, der einen Kontrast mit dem Fasermaterial **990** an bitten kann, wie eine schwarz glänzende Fläche, vorzugsweise als Andruckfläche z.B. und mit einer Schicht aus geringen Reibungskoeffizienten und/oder aus Stahl umfassen.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

## Patentansprüche

1. Prüfeinrichtung (100) zum Prüfen von aus einer Mündung tretenden Fasermaterial (990) einer Fasermasse auf dessen Kennwerte; Fasermaterialen (990) können Fasern, Teile von Fasern und Verunreinigungen, wie Trash, Staub, Pflanzenrückstände und andere Verschmutzungen umfassen, und können, beispielsweise einem sogenannten Streckenband, Kardenband oder Teilen davon, mittels einer sich drehenden Speisewalze einer drehenden Auflösewalze zugeführt werden; die Prüfeinrichtung (100) umfassend:
- eine Sammelfläche (110); die Sammelfläche (110) ist so ausgelegt, dass sie das Fasermaterial (990) sammelt;
- eine Andruckfläche (120); die Andruckfläche (120) ist so ausgelegt, dass das gesammelte Fasermaterial (990) gedrückt (520) ist, um die Eigenschaften des gesammelten Fasermaterials (990) zu erfassen (530);
- wenigstens einen Stellmotor (131, 132); der wenigstens eine Stellmotor (131, 132) ist so ausgelegt, dass er wenigstens eine Fläche (110, 120), aus Sammelfläche (110) und Andruckfläche (120), zwischen wenigstens einer ersten Position (111), wobei das gesammelte Fasermaterial (990) zwischen der Andruckfläche (120) und der Sammelfläche (110) gedrückt (520) ist, und wenigstens einer zweiten Position (112), wobei das gesammelte Fasermaterial (990) befreit (540) wird und von Prüfeinrichtung (100) teilweise oder ganz entfernt wird, bewegt (551, 552).

2. Prüfeinrichtung (100) gemäss Anspruch 1, wobei der wenigstens einen Stellmotor (131, 132) umfasst, wenigstens einen ersten Stellmotor (131), der konfiguriert ist, die Sammelfläche (110) zu bewegen, und/oder wenigstens einen zweiten Stellmotor (132), der konfiguriert ist, die Andruckfläche (120) zu bewegen.

3. Prüfeinrichtung (100) gemäss Anspruch 1 oder 2, welche wenigstens eine Putzeinrichtung (141, 142) umfasst; die wenigstens eine Putzeinrichtung (141, 142) so ausgelegt ist, dass sie die wenigstens eine Fläche (110, 120), aus Sammelfläche (110) und Andruckfläche (120), putzt, vorzugsweise, wenn die wenigstens eine Fläche (110, 120), aus Sammelfläche (110) und Andruckfläche (120), zwischen der wenigstens einen ersten Position (111) und der wenigstens einen zweiten Position (112) bewegt (551, 552) wird.

4. Prüfeinrichtung (100) gemäss Anspruch 3, wobei die wenigstens eine Putzeinrichtung (141, 142) wenigstens eine erste Putzeinrichtung (141) und/oder wenigstens eine zweite Putzeinrichtung (142) umfasst; die wenigstens eine erste Putzeinrichtung (141) ist so konfiguriert, um die Sammelfläche (110) zu putzen (540), und die wenigstens eine zweite Putzeinrichtung (142) ist so konfiguriert, um die Andruckfläche (120) zu putzen (540).

5. Prüfeinrichtung (100) gemäss irgendeinem der Ansprüche 1 bis 4, wobei die Sammelfläche (110) eine Platte oder ein Riemen ist, und/oder die Andruckfläche (120) ein Riemen oder eine Platte ist.

6. Prüfeinrichtung (100) gemäss irgendeinem der Ansprüche 1 bis 5, wobei die Sammelfläche (110) und/oder die Andruckfläche (120) teilweise oder ganz eine Schicht aus geringen Reibungskoeffizienten, vorzugsweise aus Polytetrafluorethylen, umfasst.

7. Prüfeinrichtung (100) gemäss irgendeinem der Ansprüche 1 bis 6, wobei die Sammelfläche (110) und/oder die Andruckfläche (120) teilweise oder ganz aus Stahl, vorzugsweise aus Edelstahl, besteht.

8. Prüfeinrichtung (100) gemäss irgendeinem der Ansprüche 1 bis 7, wobei die Sammelfläche (110) ganz oder teilweise einen transparenten Bereich zum Erfassen (530) der Eigenschaften des gesammelten Fasermaterials (990) umfasst.

9. Prüfeinrichtung (100) gemäss irgendeinem der Ansprüche 1 bis 8, welche eine Analyseeinrichtung (150) umfasst, die konfiguriert ist, die die Eigenschaften des gesammelten Fasermaterials (990) zu erfassen (530), vorzugsweise in der wenigstens einen ersten Position (111).

10. Prüfeinrichtung (100) gemäss Anspruch 9, wobei die Analyseeinrichtung (150) einen Sender (151) und/oder einen Empfänger (152) umfasst; der Sender (151) ist so ausgelegt, dass er das gesammelte Fasermaterial (990) strahlt, und/oder der Empfänger (152) ist so ausgelegt, dass er teilweise oder ganz die reflektierten elektromagnetischen Wellen und/oder die durchgedrungenen elektromagnetischen Wellen empfängt.

11. Prüfeinrichtung (100) gemäss Anspruch 10, wobei die Analyseeinrichtung (150) eine Kamera (156), ein Ultraschallgerät (157) oder ein Röntgengerät (158) umfasst.

12. Prüfverfahren (500) zum Prüfen von Fasermaterial (990) auf dessen Kennwerte, das von der Prüfeinrichtung (100) gemäss irgendeinem der Ansprüche 1 bis 11 implementierte ist; das Prüfverfahren (500) umfassend:
- eine Sammlung (510) von Fasermaterialen (990) auf einer Sammelfläche (110);
- ein Druck (520) des gesammelten Fasermaterials (990) zwischen einer Andruckfläche (120) und der Sammelfläche (110);
- eine Erfassung (530) der Eigenschaften des gesammelten Fasermaterials (990); und,
- eine Befreiung (540) der Sammelfläche (110) und der Andruckfläche (120) von Fasermaterial (990).

13. Prüfverfahren (500) gemäss Anspruch 12, welches eine Bewegung (551, 552) der wenigstens einen Fläche (110, 120), aus Sammelfläche (110) und Andruckfläche (120), umfasst, zwischen wenigstens einer ersten Position (111), wobei das gesammelte Fasermaterial (990) zwischen der Andruckfläche (120) und der Sammelfläche (110) gedrückt (520) ist, und wenigstens einer zweiten Position (112), wobei die Sammelfläche (110) und die Andruckfläche (120) vom gesammelten Fasermaterial (990) befreit (540) werden.

14. Prüfverfahren (500) gemäss Anspruch 13, wobei die Erfassung (530) stattfindet, während der in wenigstens einer ersten Position (111) sich die wenigstens eine Fläche (110, 120) befindet.

15. Prüfverfahren (500) gemäss Anspruch 13 oder 14, wobei die Befreiung (540) stattfindet, während der in wenigstens einer zweiten Position (112) sich die wenigstens eine Fläche (110, 120) befindet.
